# DEMANDE DE BREVET EUROPEEN

(11) **EP 2 607 339 A1**
(43) Date de publication de la demande: **26.06.2013**
(21) Numéro de dépôt: 12290402.2
(22) Date de dépôt: 20.11.2012
(51) Int. Cl.: C07C 2/08, C07C 11/08, C07C 11/02

(54) **Procédé de production d'octènes mettant en oeuvre la dimérisation de l'éthlène en butènes et la dimérisation des butènes en octènes**

(30) Priorité: 20.12.2011 FR 1103996
(71) Demandeur: IFP Energies nouvelles, 92852 Rueil-Malmaison Cedex (FR)
(72) Inventeur: Olivier-Bourbigou, Hélène, 69230 Saint Genis-Laval (FR); Hugues, Francois, 69390 Charly, Vernaison (FR); Magna, Lionel, 69007 Lyon (FR); Breuil, Pierre-Alain, 69006 Lyon (FR)

(57) **Abrégé**

La présente invention décrit un procédé de production d'octènes à partir d'éthylène mettant en oeuvre la dimérisation de l'éthylène en butènes et la dimérisation des butènes en octènes.

## Description

La présente invention décrit un procédé de production d'octènes à partir d'éthylène mettant en oeuvre la dimérisation de l'éthylène en butènes et la dimérisation des butènes en octènes.

### Art antérieur sur la production d'octènes

Il existe différentes sources de production d'octènes :
i) l'extraction d'une coupe oléfinique C8 d'un procédé type polygas (procédé Cat Poly™) de C4 de craquage catalytique,
ii) la dimérisation des n-butènes contenus dans un Raffinat Il par un procédé de type Dimersol™ ou Octol™,
iii) la dimérisation de l'isobutène contenu dans un Raffinat I par un procédé de type SeleCtopol™ ou Dimer8™ ou NextOctane™ ou In Alk™.

Les voies i) et iii) conduisent à l'obtention d'oléfines en C8 très ramifiées. Les octènes produits par la voie ii) de dimérisation des n-butènes sont particulièrement intéressants car ils sont peu ramifiés.

Ces octènes produits par le procédé Dimersol™ d'Axens (Revue de l'Institut Français du Pétrole, Vol. 37, N°5, septembre-octobre 1982, p 639) ou le procédé Octol™ de Hüls AG (Hydrocarbon Processing, Février 1992, p 45-46), obtenus à des coûts avantageux, peuvent en particulier être transformés avec de bons rendements par réaction d'hydroformylation puis d'hydrogénation en isononanols. Ces alcools en C9 sont notamment utilisés pour la synthèse de plastifiants de type phtalates pour le PVC. Ces alcools confèrent aux polymères des propriétés particulières : très bonne compatibilité avec la chaîne polymère, faible volatilité, flexibilité à basse température, stabilité à haute température, processabilité ...

Selon cette voie ii) les charges utilisées sont des coupes C4. Les coupes C4 sont disponibles en grande quantité dans les raffineries et les sites pétrochimiques. Elles sont produites par les procédés classiques tels que le craquage catalytique en lit fluidisé ou le craquage du naphta à la vapeur. Ces coupes C4 contiennent typiquement des composés oléfiniques tels que des n-butènes (butène-1 et butènes-2), de l'isobutène et également des paraffines (isobutane et n-butane), des diènes (tel que le butadiène-1,3) ainsi que des traces d'impuretés acétyléniques. Le Tableau 1 montre les compositions types de coupes C4 issues d'un craquage catalytique à lit fluidisé et d'un craquage à la vapeur.

Effectués sur ces coupes C4 oléfiniques, les procédés de dimérisation nécessitent a) l'élimination du butadiène par extraction au solvant ou hydrogénation sélective en butènes et b) la séparation ou la transformation de l'isobutène.

**Tableau 1 : Exemple de composition types de coupe C4**

| | Composition typique (%pds) FCC (après hydrogénation sélective) | Composition (%pds) Craquage à la vapeur (après hydrogénation sélective) |
|---|---|---|
| n-Butane | 11 | 5 |
| Butène-1 | 13 | 28 |
| Butène-2 | 26 | 20 |
| Isobutène | 15-25 | 45-50 |
| Isobutane | 35 | 2 |
| Butadiene | 0,5 | 1 |

Industriellement, la séparation du butadiène se fait par exemple par extraction liquide-liquide avec un solvant (N-méthyl-2-pyrrolidone, DMF, ...) et/ou par hydrogénation sélective permettant d'obtenir un mélange d'isobutènes, de n-butènes et de butanes. L'isobutène est ensuite séparé des autres composés C4. La séparation de l'isobutène est réalisée par des voies détournées en le transformant par des procédés tels que l'éthérification par le méthanol ou l'oligomérisation sélective ou la polymérisation conduisant respectivement le plus typiquement au MTBE (méthyl-tert-butyl-éther), au DIB (diisobutène) ou PIB (polyisobutène). Après élimination de l'isobutène, il ne reste que les n-butènes et les butanes (Raffinat II). La publication (Petroleum Technolgoy Quartely, autumn 1999, p 141) décrit cet enchaînement d'étapes pour la production d'octènes à partir de coupes C4.

Une possibilité supplémentaire pour transformer les oléfines C4 en octènes est l'enchaînement des procédés Dimersol™-Difasol™ ou l'utilisation directe du procédé biphasique Difasol™ tels que décrits dans l'article Oil Gas Eur. Mag. 2005, 2, p 83.

Cependant, il est connu (Revue de l'IFP, volume 37, N°5 sept-oct. 1982 p 639) que l'isobutène, oléfine ramifiée, est susceptible de réagir au cours du procédé de dimérisation, selon un processus cationique pour former des oligomères lourds. Les hydrocarbures polyinsaturés (diènes) sont des poisons pour la dimérisation. Les composés polaires azotés, oxygénés ou soufrés (qui peuvent venir des étapes d'extraction du butadiène par des solvants polaires ou des étapes d'éthérification pour séparer l'isobutène) sont également des poisons des catalyseurs même s'ils sont présents à l'état de traces.

L'utilisation de ces coupes C4 nécessite en conséquence des étapes de purification. Les traces d'impuretés citées ci-dessus ne sont en général jamais complètement éliminées.

### Résumé de l'invention

La présente invention décrit un procédé de production d'octènes à partir d'éthylène mettant en oeuvre les étapes suivantes :
a) la dimérisation de l'éthylène en butènes en présence d'un catalyseur,
b) la dimérisation des butènes obtenus à l'étape a) en octènes en présence d'un catalyseur.

### Avantage de l'invention

On a maintenant découvert qu'il pouvait être avantageux de former les butènes pour la dimérisation par dimérisation de l'éthylène.

Un des premiers avantages du procédé de l'invention par rapport aux procédés utilisant une coupe C4 du craquage catalytique en lit fluidisé ou du craquage du naphta à la vapeur est qu'il n'est pas besoin d'effectuer de purification. En particulier, on n'a pas à éliminer l'isobutène puisque les n-butènes issus de la dimérisation n'en contiennent pas. En effet, le procédé selon la présente invention peut être effectué sans étape de purification entre l'étape de dimérisation de l'éthylène et l'étape de dimérisation des butènes.

De plus, les butènes obtenus par dimérisation de l'éthylène sont exempts de butane, ce qui a l'avantage de ne pas diluer le réactif oléfinique dans la charge du procédé de dimérisation.

Par ailleurs, les butènes formés par dimérisation ne contiennent pas d'impuretés oxygénés. Leur purification n'est donc pas nécessaire. Cette absence est particulièrement intéressante pour mettre en oeuvre le procédé Dimersol™, le procédé Difasol™ seul ou en combinaison avec le procédé Dimersol™, ou le procédé Octol™.

Un autre intérêt du procédé selon l'invention, est que l'on utilise de l'éthylène (par exemple issu d'un craquage à la vapeur de l'éthane) sans qu'on soit limité par la disponibilité de la coupe C4 (Par ailleurs, un autre intérêt est que l'on peut utiliser une autre source d'éthylène que l'origine fossile. En particulier, l'éthylène peut provenir de ressources renouvelables, par exemple de déshydratation d'éthanol, ou tout autre source. L'éthylène est ainsi issu d'une ressource fossile et/ou d'une ressource renouvelable.

### Description du procédé

La première étape du procédé selon l'invention comprend la dimérisation de l'éthylène en butènes en présence d'un catalyseur.

### a/ Dimérisation de l'éthylène en butènes

La dimérisation de l'éthylène en butènes peut être réalisée par tout procédé catalytique connu de l'art antérieur, et parmi ceux ci on choisira de préférence ceux qui conduisent à une forte sélectivité en dimères (butène-1 ou -2).

Selon une première variante, la dimérisation de l'éthylène est mise en oeuvre en présence d'un catalyseur à base de titane. De tels catalyseurs sont décrits dans le brevet FR2552079 (US4615998). On utilise un système catalytique homogène apte à produire sélectivement du butène-1 à partir d'éthylène dans des conditions peu sévères. Le système catalytique est composé d'un titanate d'alkyle, d'un additif organique de type éther et d'un composé de l'aluminium de formule AlR₃ ou AlR₂H dans laquelle chacun des restes R est un radical hydrocarbyle, de préférence alkyle comprenant de 2 à 6 atomes de carbone, par exemple le triéthylaluminium, le tripropylaluminium, le triisobutylaluminium, l'hydrure de diisobutylaluminium et le trihexylaluminium. Les titanates d'alkyle utilisés répondent à la formule générale Ti(OR')₄ dans laquelle R' est un radical alkyle linéaire ou ramifié comportant de 2 à 8 atomes de carbone, par exemple le titanate de tétraéthyle, le titanate de tétraisopropyle, le titanate de tétra-n-butyle et le titanate de tétra-2-éthyl-hexyle. L'additif organique de type éther et le titanate d'alkyle sont mis en oeuvre dans un rapport molaire de 1:0.5 a 10:1. La réaction de dimérisation est mise en oeuvre à une température entre 20 et 70°C. La pression est de préférence de 0.5 à 8 MPa.
Le procédé Alphabutol™ (décrit dans la revue Hydrocarbon Processing, 1984 pp 118-120) est basé sur cette technologie et conduit à la production industrielle de butène-1 selon un schéma simple.

Selon une deuxième variante, la dimérisation de l'éthylène est mise en oeuvre en présence d'un système catalytique composé d'un composé du nickel et d'un composé d'aluminium. De tels systèmes catalytiques sont décrits dans le brevet US3485881. Ce procédé consiste à dimériser des monooléfines (dont de l'éthylène) en les mettant en contact avec un système catalytique formé par le mélange d'un premier composé sélectionné dans le groupe comprenant les formules suivantes : (R₃P)₂NiY₂, (R₃PO)₂NiX₂, (R₃AsO)₂NiX₂, (pyridine)₂NiX₂, (bipyridine)NiX₂, (phénanthroline)NiX₂ et un complexe formé par un composé azoté bicyclique avec NiX₂, où R est un groupement hydrocarboné ayant jusqu'à 20 atomes de carbone, X est un halogène, Y est choisi parmi les halogènes ou les groupements hydrocarbonés comme définis ci-dessus et la pyridine, la bipyridine et la phénanthroline peuvent être substitués ou non avec un ou des groupements hydrocarbonés, et un second composé représenté par la formule R'_{z}AlX_{y} où x et y sont des nombres entiers entre 1 et 3, R' est un groupement hydrocarboné ayant jusqu'à 20 atomes de carbone et X est un halogène. Le ratio entre le premier composé (Ni) et le deuxième composé (Al) est entre 1:0,5 et 1:20. La réaction de dimérisation est mise en oeuvre à une température entre -80 °C et 100 °C. La pression peut aller jusqu'à 13,8 MPa (2000 psig). On obtient des butènes d'une sélectivité supérieure à 81 %

La pureté très élevée des butènes issus des procédés de dimérisation de l'éthylène selon l'invention permet de s'affranchir d'une étape préalable de purification entre l'étape de dimérisation de l'éthylène et l'étape de dimérisation des butènes.

En effet, l'effluent de la dimérisation de l'éthylène contient principalement des butènes, ainsi que des faibles quantités d'éthylène non réagi et d'oligomères (notamment du C6 et C8). Ces effluents sont notamment exempts de tout composé susceptible d'empoisonner le catalyseur de la dimérisation suivante des butènes.

Selon une première variante, l'effluent obtenu après la dimérisation de l'éthylène et contenant les butènes, et après avoir séparé optionnellement le catalyseur, est envoyé tel quel dans l'étape de dimérisation des butènes. Les oligomères présents dans les butènes sont inertes dans la dimérisation des butènes.

Selon une deuxième variante, l'effluent obtenu après la dimérisation de l'éthylène et contenant les butènes, et après avoir séparé optionnellement le catalyseur, est envoyé dans une section de séparation permettant de séparer les butènes de l'éthylène non réagit d'une part (qui est recyclé au réacteur de dimérisation de l'éthylène), des oligomères formés lors de la dimérisation d'éthylène (hexènes et octènes) d'autre part. Cette séparation peut être effectuée par une distillation ou une séparation de type flash.

La deuxième étape du procédé selon l'invention comprend la dimérisation des butènes obtenus à l'étape a) en octènes en présence d'un catalyseur.

### b/ Dimérisation des n-butènes en octènes

La dimérisation des butènes en octènes peut être réalisée par tout procédé catalytique connu de l'art antérieur, et parmi ceux ci on choisira de préférence ceux qui conduisent à une forte sélectivité en dimères (octènes linéaires ou faiblement ramifiés).

Selon une première variante, la dimérisation des butènes est mise en oeuvre en présence d'un système catalytique en phase liquide comprenant un composé du nickel et d'un composé d'aluminium. De tels systèmes catalytiques sont décrits pour le procédé Dimersol™ commercialisé par la société Axens. On utilisera les diverses compositions catalytiques décrites pour le procédé selon l'invention.

Par exemple, le brevet FR 2 443 877 décrit une composition comprenant : au moins un composé de nickel bivalent, au moins un dihalogénure d'hydrocarbylaluminium répondant à la formule AlRₙX₃₋ₙ dans laquelle R est un radical hydrocarbyle comprenant de 1 à 12 atomes de carbone, X est un atome de chlore ou de brome, et n est un nombre compris entre 0 et 1.

Une composition catalytique améliorée est décrite dans le brevet FR2794038. Elle est obtenue par mise en contact, dans un ordre quelconque : d'au moins un composé de nickel bivalent, avec au moins un dihalogénure d'hydrocarbylaluminium de formule AlRX₂ dans laquelle R est un radical hydrocarbyle comprenant de 1 à 12 atomes de carbone, X est un atome de chlore ou de brome, et au moins un acide organique de Bronsted, ayant de préférence un pKa au plus égal à 3.

Comme composé du nickel bivalent on peut utiliser tous les composés solubles à plus de 1 g par litre en milieu hydrocarboné, et plus particulièrement dans les réactifs et le milieu de réaction. On utilise de préférence les carboxylates de nickel de formule générale (R₁ COO)₂Ni, où R₁ est un reste hydrocarbyle, par exemple alkyle, cycloalkyle, alkényle, aryle, aralkyle ou alkaryle, contenant jusqu'à 20 atomes de carbone, de préférence un reste hydrocarbyle de 5 à 20 atomes de carbone. Le radical R₁ peut être substitué par un ou plusieurs atomes d'halogène, groupes hydroxy, cétone, nitro, cyano ou autres groupes qui ne gênent pas la réaction. Les deux radicaux R₁ peuvent aussi constituer un reste alkylène de 6 à 18 atomes de carbone. Des exemples, non limitatifs, de composés du nickel sont les sels de nickel bivalent suivants : octoate, éthyl-2 hexanoate, décanoate, stéarate, oléate, salicylate, hydroxydécanoate. On utilise de préférence l'éthyl-2 hexanoate de nickel.

Le composé acide de Bronsted répond à la formule HY, où Y est un anion organique, par exemple carboxylique, sulfonique ou phénolique. On préfère les acides dont le pKₐ à 20 °C est au maximum égal à 3, plus particulièrement ceux qui sont de plus solubles dans le composé de nickel ou dans sa solution dans un hydrocarbure ou un autre solvant convenable. Une classe préférée d'acides comprend le groupe formé par les acides halogénocarboxyliques de formule R₂COOH dans laquelle R₂ est un radical alkyle halogéné, notamment ceux qui renferment au moins un atome d'halogène en alpha du groupe -COOH avec au total de 2 à 10 atomes de carbone. On utilise de préférence un acide halogénoacétique de formule CXₚH₃-p-COOH dans laquelle X est le fluor, le chore, le brome ou l'iode, avec p entier de 1 à 3. A titre d'exemple, on peut citer les acides trifluoroacétique, difluoroacétique, fluoroacétique, trichloroacétique, dichoroacétique, chloroacétique. Ces exemples ne sont pas limitatifs, et on peut aussi utiliser les acides arylsulfoniques, alkylsulfoniques, fluoroalkylsulfoniques, l'acide picrique, l'acide nitroacétique. On utilise de préférence l'acide trifluoroacétique.

Les composés de dihalogénure d'hydrocarbylaluminium enrichis en trihalogénure d'aluminium répondent à la formule générale AlRₙX₃₋ₙ dans laquelle R est un radical hydrocarbyle comprenant de 1 à 12 atomes de carbone, tel que alkyle, aryle, aralkyle, alkaryle ou cycloalkyle, X est un atome de chlore ou de brome, et n est un nombre compris entre 0 et 1. Ils sont obtenus par mélange d'un dihalogénure d'hydrocarbylaluminium de formule AlRX₂ dans laquelle R est un radical hydrocarbyle comprenant de 1 à 12 atomes de carbone, tel que alkyle, aryle, aralkyle, alkaryle ou cycloalkyle et X est un atome de chlore ou de brome, avec un trihalogénure d'aluminium AlX₃. Sans que la liste suivante soit limitative, on peut citer à titre d'exemple de tels composés : le dichloroéthylaluminium enrichi avec du chlorure d'aluminium, le mélange ayant une formule AlEt_{0,9}Cl_{2,1}, le dichloroisobutylaluminium enrichi avec du chlorure d'aluminium, le mélange ayant une formule AliBu_{0,9}Cl_{2,1}, le dibromoéthylaluminium enrichi avec du bromure d'aluminium, le mélange ayant une formule AliBu_{0,9}Cl_{2,1}.

De manière optionnelle, les composants décrits ci-dessus sont mélangés, dans un solvant, à une température contrôlée et pendant un temps déterminé, qui constitue une étape dite de pré-conditionnement avant leur utilisation dans la réaction tel que décrit dans le brevet US7235703. Plus précisément, ce pré-conditionnement de la composition catalytique consiste à effectuer le mélange des trois composants dans un solvant hydrocarboné, par exemple un alcane ou un hydrocarbure aromatique, ou encore un hydrocarbure halogéné, ou encore de façon préférée les oléfines produites dans la réaction d'oligomérisation, sous agitation et sous atmosphère inerte, par exemple sous azote ou sous argon, à une température contrôlée comprise entre 0 et 80°C, de préférence entre 10 et 60°C, pendant une durée de 1 minute à 5 heures, de préférence de 5 minutes à 1 heure. La solution ainsi obtenue est ensuite transférée sous atmosphère inerte dans le réacteur de dimérisation.

Une autre composition améliorée est décrite dans le brevet US 4283586. Elle comprend les composés du nickel et de l'aluminium décrits précédemment ainsi qu'un polyol organique tel que l'éthylène glycol.

Le procédé Dimersol™ est en général réalisé à une température de -20°C à 80°C, dans des conditions de pression telles que les réactifs soient maintenus au moins en majeure partie en phase liquide ou en phase condensé.

L'étape de dimérisation peut être mise en oeuvre dans un réacteur à un ou plusieurs étages de réaction en série, la charge oléfinique et/ou la composition catalytique au préalable pré-conditionnée étant introduits en continu, soit dans le premier étage, soit dans le premier et un autre quelconque des étages. A la sortie du réacteur, le catalyseur peut être désactivé, par exemple par injection d'ammoniac et/ou d'une solution aqueuse de soude et/ou d'une solution aqueuse d'acide sulfurique. Les oléfines non converties et les alcanes éventuellement présents dans la charge sont ensuite séparés des oligomères par distillation.

Selon une deuxième variante, la dimérisation des butènes est mise en oeuvre en présence d'un système catalytique comprenant un complexe de nickel ou un mélange de complexes de nickel en milieu biphasique liquide-liquide contenant un milieu à caractère ionique pas ou peu miscible avec la phase organique contenant les octènes. Un tel système catalytique est décrit dans le brevet US 5104840 (procédé Difasol™).

Le milieu à caractère ionique comprend au moins un sel de formule Q⁺A⁻, dans lequel Q⁺ est un cation ammonium ou phosphonium quaternaire ou un mélange des deux ou un cation lithium et A- est un anion coordinant ou non-coordinant, choisi dans le groupe formé par les halogénoaluminates, les organohalogénoaluminates, les organogallates, les organohalogénogallates ou un mélange d'au moins deux de ces composés.

Ainsi, les catalyseurs utilisables sont des complexes quelconques du nickel, mis à part les complexes du nickel solvatés par des composés à protons actifs tels que l'eau, les alcools, les acides organiques, l'ammoniac. A titre d'exemples de tels complexes, on peut citer l'acétylacétonate de nickel, les complexes des halogénures ou du sulfate de nickel tels que (NiCl₂, 2 pyridine), (NiCl₂, 2PPr₃), (NiBr₂, 2PBu₃), (NiSO₄, 2PBu₃), l'octoate de nickel, les alcoolates de nickel. On peut aussi utiliser les composés du nickel décrits dans le brevet français FR2611700, qui comportent au moins une liaison nickel-carbone ou nickel-hydrogène, ou qui sont à une valence inférieure à 2 ; à titre d'exemples de tels composés, on peut citer le biscyclooctadiène nickel, le chlorure de méthallylnickel, le bisallylnickel, le chlorure d'allylnickel, le bromure d'allylnickel.

La réaction catalytique en milieu biphasique est effectuée à une température inférieure à +100°C, par exemple entre -50°C et +100°C, la pression sera maintenue entre 0,01 et 20 MPa.

La mise en oeuvre en système biphasique permet, par rapport au système liquide tout homogène tel que décrit dans le procédé Dimersol™, de réduire le volume réactionnel ainsi que la consommation de catalyseur et d'augmenter la sélectivité en dimères.

Selon une troisième variante, le système biphasique Difasol™ peut également être mis en oeuvre après une première étape homogène Dimersol™ tel que décrit dans les brevets US 6444866 et US 6203712. Dans ce cas, la dimérisation des butènes comporte au moins deux étapes successives, la première étant mise en oeuvre en présence d'un système catalytique en phase liquide comprenant un composé du nickel et un composé d'aluminium, la deuxième étant mise en oeuvre en présence d'un système catalytique comprenant un complexe de nickel ou un mélange de complexes de nickel en milieu biphasique liquide-liquide contenant un milieu à caractère ionique pas ou peu miscible avec la phase organique contenant les produits de réaction (octènes).

La composition catalytique de la première étape, en phase homogène liquide est de préférence la suivante : le catalyseur est un composé du nickel ou un mélange de composés du nickel, le co-catalyseur est un alkyle aluminium ou un mélange d'alkyles aluminium ou un halogénoalkyle aluminium ou un mélange d'halogénoalkyles aluminium, l'acide de Bronsted est un acide halogénoacétique ou un mélange d'acides halogénoacétiques et l'éventuel additif de catalyseur peut être un composé à caractère acide, l'anion correspondant à cet acide, un ester d'acide carboxylique, un époxy ou une phosphine.

La composition catalytique de la deuxième étape est identique à celle décrite ci-dessus pour le procédé Difasol™. La phase polaire de la second étape peut aussi être un milieu ionique non miscible avec la phase organique ne contenant pas de catalyseur, le catalyseur de la deuxième étape en milieu biphasique liquide-liquide est alors le catalyseur utilisé en première étape. Le catalyseur est alors introduit dans le réacteur avec l'effluent de sortie du réacteur de la première étape.

Les conditions opératoires des deux étapes sont identiques à celles indiquées ci-dessus pour le procédé Dimersol™ et Difasol™, respectivement.

Selon une quatrième variante, la dimérisation des butènes est mise en oeuvre en présence d'un catalyseur hétérogène contenant des oxydes du nickel, du silicium et de l'aluminium. Un tel catalyseur est décrit dans les brevets US5177282 et DD160037 (procédé Octol™).

Cette variante consiste à dimériser des butènes avec un catalyseur hétérogène à base de nickel à des températures comprises entre 0°C et 200°C et des pressions comprises entre 0,1 et 7 MPa.

De manière préférée, les butènes sont dimérisés dans un réacteur à lit fixe sur un catalyseur hétérogène contenant des oxydes du nickel, du silicium et de l'aluminium. Les butènes sont dimérisés dans la phase liquide.

Le catalyseur contient de manière préférée des oxydes du nickel, du silicium et de l'aluminium. De manière très préférée, le catalyseur contient entre 0,1 et 10 % poids d'alumine et entre 0,1 et 10 % poids d'oxyde de nickel, et de la silice.

Selon une cinquième variante, la dimérisation des butènes est mise en oeuvre en présence d'un catalyseur hétérogène contenant du nickel, ainsi que du chlore ou du soufre, déposés sur alumine. Un tel procédé est décrit dans les brevets EP346187, EP272970 et FR2641477. Il conduit à la formation sélective de dimères à partir d'oléfines légères.

Selon cette variante, la dimérisation des butènes peut être effectuée en phase liquide en présence d'au moins un catalyseur de dimérisation d'oléfines renfermant du chlore et du nickel (dont les proportions sont de préférence non-stoechiométriques) déposés sur alumine, la teneur pondérale en nickel dudit catalyseur étant en général comprise entre 0,5 et 15 % et la teneur pondérale en chlore dudit catalyseur étant habituellement comprise entre 0,4 et 15%. Le procédé est effectué à une température entre 10 et 150°C, la pression est maintenue de telle façon que le mélange réactionnel soit à l'état liquide.

Dans le cas d'un catalyseur hétérogène contenant du nickel et du soufre déposée sur alumine, on obtient la composition catalytique en opérant de la façon suivante :
a) on met en contact de l'alumine avec une source d'ions sulfate et une source d'ions nickel en solution aqueuse ou organique, les proportions d'ions sulfate et d'ions nickel étant choisies telles que l'on introduit les-dits ions dans l'alumine en proportions correspondant à un rapport atomique S : Ni inférieur à 1 : 1 et de préférence compris entre 0,1 : 1 et 0,95 :1,
b) on élimine le solvant de d'alumine qui a absorbé les-dits ions dans les proportions ci-dessus et,
c) on la chauffe entre 300 et 800°C en atmosphère non réductrice.

La pureté très élevée des butènes issus des procédés de dimérisation de l'éthylène selon l'invention permet de s'affranchir d'une étape de purification entre l'étape de dimérisation de l'éthylène et l'étape de dimérisation des butènes.

En effet, des étapes de purification sont préconisées quand il s'agit d'oléfines C4 en provenance du raffinage. Par exemple, dans le cas des procédés de dimérisation hétérogène (cas du nickel), les oléfines de la charge sont passées sur un tamis moléculaire ayant un diamètre de pores entre 4 et 15 angströms pour capter les poisons du catalyseur : l'eau, les alcools, les composés azotés, les composés soufrés ou les composés halogénés.

Les produits du présent procédé (octènes) peuvent trouver une application par exemple comme composant de carburants pour automobile et/ou comme charge dans un procédé d'hydroformylation pour la synthèse d'aldéhydes et d'alcools.

### Exemple

L'exemple qui suit illustre l'invention sans en limiter la portée.

### Etape a) Production de butène-1 par dimérisation de l'éthylène

Un flux d'éthylène pur de 3,2 t/h, issu d'une unité de craquage à la vapeur d'éthane, est envoyé dans un réacteur opérant essentiellement en phase liquide, dans lequel est envoyée 10, 8 kg/h d'une solution à 6% pds de triéthylaluminium dans l'hexane, ainsi que 1,3 kg/h d'un catalyseur liquide à base de titane. Le réacteur est maintenu à une température de 50 à 55 °C par enlèvement continu de la chaleur de réaction au moyen d'une boucle de recirculation munie d'un échangeur à eau froide, et à une pression de 2,4 MPa. Dans ces conditions, la conversion de l'éthylène par passe est de 85 %. L'effluent de sortie du réacteur est neutralisé par une base et le catalyseur est séparé des produits de la réaction. Les produits sont ensuite séparés par deux colonnes de distillation en :
- éthylène non réagi, qui est recyclé à l'entrée du réacteur,
- coupe C4, constituée essentiellement de butène-1 pur à plus de 99, 5 %, à un débit de 2,9 t/h,
- coupe C6, constituée d'un mélange d'isomères constitués majoritairement d'éthyl-2-butène-1 et de méthyl-3-pentène-1 à un débit de 0,22 t/h.

### Etape b) Production d'octènes par dimérisation du butène-1

La coupe C4 obtenue dans l'étape a) est envoyée dans une unité constituée de trois réacteurs disposés en série, opérant en phase liquide, tous équipés d'une boucle de recirculation munie d'un échangeur à eau froide, afin d'évacuer en continu la chaleur de réaction, et maintenir la température entre 45 et 50 °C.

La coupe C4 (2,9 t/h) est injectée dans le 1^{er} réacteur, ainsi que 11,6 kg/h d'une solution de dichloroalkylaluminium à 50% pds dans une coupe essence paraffinique C6-C8, et 1,9 kg/h de catalyseur liquide à base de nickel.

L'effluent du 1^{er} réacteur est intégralement envoyé dans le second, l'effluent du 2^{nd} réacteur dans le troisième.

Dans ces conditions, la conversion du butène est de 81 %.

L'effluent de sortie du troisième réacteur est neutralisé par une base et le catalyseur est séparé des produits de réaction; les produits sont séparés par deux colonnes de distillation en :
- coupe C4, à un débit de 0,56 t/h - contenant le butène n'ayant pas réagi - qui est envoyée au pool GPL (gaz de pétrole liquéfié),
- coupe C8, à un débit de 1,95 t/h, constituée d'un mélange d'isomères (n-octènes : 5% ; Methylheptènes : 65 %, Diméthylhexènes : 30%),
- coupe C12 + à un débit de 0,39 t/h, constituée majoritairement de dodécènes.

## Revendications

1. Procédé de production d'octènes à partir d'éthylène mettant en oeuvre les étapes suivantes :
a) la dimérisation de l'éthylène en butènes en présence d'un catalyseur,
b) la dimérisation des butènes obtenus à l'étape a) en octènes en présence d'un catalyseur.

2. Procédé selon la revendication précédente dans lequel l'effluent obtenu après la dimérisation de l'éthylène et contenant les butènes, est envoyé tel quel dans l'étape de dimérisation des butènes.

3. Procédé selon la revendication 1 dans lequel l'effluent obtenu après la dimérisation de l'éthylène et contenant les butènes, est envoyé dans une section de séparation permettant de séparer les butènes de l'éthylène non réagit d'une part, des oligomères formés lors de la dimérisation de l'éthylène d'autre part.

4. Procédé selon les revendications précédentes dans lequel la dimérisation de l'éthylène est mise en oeuvre en présence d'un catalyseur à base de titane.

5. Procédé selon la revendication précédente dans lequel le catalyseur à base de titane est composé d'un titanate d'alkyle, d'un additif organique de type éther et d'un composé de l'aluminium de formule AlR₃ ou AlR₂H dans laquelle chacun des restes R est un radical hydrocarbyle, et l'additif de type éthers et le titanate d'alkyle sont mis en oeuvre dans un rapport molaire de 1:0.5 a 10:1.

6. Procédé selon les revendications 1 à 3 dans lequel la dimérisation de l'éthylène est mise en oeuvre en présence d'un système catalytique composé d'un composé du nickel et d'un composé d'aluminium.

7. Procédé selon la revendication précédente dans lequel le système catalytique est un mélange d'un premier composé sélectionné dans le groupe comprenant les formules suivantes : (R₃P)₂NiY₂, (R₃PO)₂NiX₂, (R₃AsO)₂NiX₂, (pyridine)₂NiX₂, (bipyridine)NiX₂, (phénanthroline)NiX₂ et un complexe formé par un composé azoté bicyclique avec NiX₂, où R est un groupement hydrocarboné ayant jusqu'à 20 atomes de carbone, X est un halogène, Y est choisi parmi les halogènes ou les groupements hydrocarbonés comme définis ci-dessus et la pyridine, la bipyridine et la phénanthroline peuvent être substitués ou non avec un ou des groupements hydrocarbonés, et un second composé représenté par la formule R'_{z}AlX_{y} où x et y sont des nombres entiers entre 1 et 3, R' est un groupement hydrocarboné ayant jusqu'à 20 atomes de carbone et X est un halogène.

8. Procédé selon les revendications 1 à 7 dans lequel la dimérisation des butènes est mise en oeuvre en présence d'un système catalytique en phase liquide comprenant un composé du nickel et un composé d'aluminium.

9. Procédé selon la revendication précédente dans lequel le système catalytique est composé d'au moins un composé de nickel bivalent, avec au moins un dihalogénure d'hydrocarbylaluminium de formule AlRX₂ dans laquelle R est un radical hydrocarbyle comprenant de 1 à 12 atomes de carbone, X est un atome de chlore ou de brome, et au moins un acide organique de Bronsted, ayant de préférence un pKa au plus égal à 3.

10. Procédé selon les revendications 1 à 7 dans lequel la dimérisation des butènes est mise en oeuvre en présence d'un système catalytique composé d'un complexe de nickel ou un mélange de complexes de nickel en milieu biphasique liquide-liquide contenant un milieu à caractère ionique pas ou peu miscible avec la phase organique contenant les octènes.

11. Procédé selon les revendications 1 à 7 dans lequel la dimérisation des butènes comporte au moins deux étapes successives, la première étant mise en oeuvre en présence d'un système catalytique en phase liquide comprenant un composé du nickel et un composé d'aluminium, la deuxième étant mise en oeuvre en présence d'un système catalytique comprenant un complexe de nickel ou un mélange de complexes de nickel en milieu biphasique liquide-liquide contenant un milieu à caractère ionique pas ou peu miscible avec la phase organique contenant les octènes.

12. Procédé selon les revendications 1 à 7 dans lequel la dimérisation des butènes est mise en oeuvre en présence d'un catalyseur hétérogène contenant des oxydes du nickel, du silicium et de l'aluminium.

13. Procédé selon les revendications 1 à 7 dans lequel la dimérisation des butènes est mise en oeuvre en présence d'un catalyseur hétérogène contenant du nickel, ainsi que du chlore ou du soufre, déposés sur alumine.

14. Procédé selon les revendications précédentes dans lequel l'éthylène est issu d'une ressource fossile et/ou d'une ressource renouvelable.

15. Procédé selon les revendications précédentes dans lequel l'éthylène est issu de la déshydratation d'éthanol.
